# EUROPEAN PATENT SPECIFICATION

(11) **EP 3 801 037 B1**
(45) Date of publication and mention of the grant of the patent: **19.10.2022**
(21) Application number: 19727020.0
(22) Date of filing: 29.05.2019
(51) Int. Cl.: A23C 9/13, A23C 9/142

(54) **A SIMPLE METHOD FOR THE PURIFICATION OF A SIALYLLACTOSE**
EINFACHES VERFAHREN ZUR REINIGUNG EINER SIALYLLACTOSE
PROCÉDÉ SIMPLE POUR LA PURIFICATION D'UN SIALYLLACTOSE

(30) Priority: 01.06.2018 EP 18175602
(43) Date of publication of application: 14.04.2021
(73) Proprietor: Chr. Hansen HMO GmbH, 53619 Rheinbreitbach (DE)
(72) Inventor: JENNEWEIN, Stefan, 53605 Bad Honnef (DE); HELFRICH, Markus, 53557 Bad Hönningen (DE); ENGELS, Benedikt, 53545 Linz (DE)
(86) International application number: PCT/EP2019/063946
(87) International publication number: WO 2019/229118

(56) References cited:
- EP-A1- 2 484 686
- WO-A1-2017/220697
- CN-A- 103 788 144
- US-A1- 2014 170 293
- US-B2- 8 507 227
- DROUILLARD S ET AL: "Efficient synthesis of 6'-sialyllactose, 6,6'-disialyllactose, and 6'-KDO-lactose by metabolically engineered E. coli expressing a multifunctional sialyltransferase from the Photobacterium sp. JT-ISH-224", CARBOHYDRATE RESEARCH, PERGAMON, GB, vol. 345, no. 10, 2 July 2010 (2010-07-02) , pages 1394-1399, XP027118796, ISSN: 0008-6215 [retrieved on 2010-02-24]

## Description

### Field of the invention

The present invention relates to a process for the purification of a sialyllactose. More specifically, the present invention concerns a simple and economical process for separating a sialyllactose from other carbohydrates such as lactose and monosaccharides as well as larger oligosaccharides, such as disialyllactose, which may be present as contaminating carbohydrates in a fermentation broth, if said sialyllactose is produced by microbial fermentation.

### Background

Human milk is regarded as the best diet for the development of an infant. It is composed of fats, proteins, vitamins, minerals, trace elements and complex oligosaccharides. Besides lactose, human milk - as well as milk of other mammals - contains various structurally diverse oligosaccharides which are also known as human milk oligosaccharides (HMOs) (Urashima T. et al., (2011) Milk Oligosaccharides, Nova Biomedical Books, New York ISBN 978-1-61122-831-1). Among the HMOs, sialylated HMOs (SHMOs) were observed to support the resistance to enteropathogenic bacteria and viruses. Interestingly, recent studies further demonstrated a protective effect of long-chained SHMOs against necrotizing enterocolitis, which is one of the most common and lethal diseases in preterm infants. In addition, SHMOs are believed to support an infant's brain development and its cognitive capabilities. Also, sialylated oligosaccharides have been shown to neutralize enterotoxins of various pathogenic microbes including *Escherichia coli, Vibrio cholerae* and *Salmonella.* Further, it was found that sialylated oligosaccharides interfere with the colonization of the gut by *Helicobacter pylori* and thereby prevent or inhibit gastric and duodenal ulcers.

Among the sialylated oligosaccharides, 3'-sialyllactose and 6'-sialyllactose are the most abundant members in human milk.

Since sialylated oligosaccharides have a complex structure, their chemical or (chemo) enzymatic syntheses are challenging and associated with extensive difficulties, e.g. control of stereochemistry, formation of specific linkages, availability of feedstocks, etc. As a consequence, commercially available sialylated oligosaccharides have been very expensive due to their low quantity in natural sources.

Thus, efforts in metabolic engineering of microorganisms to produce sialylated oligosaccharides have been made, since this approach is the most promising way for producing HMOs in an industrial scale. For the production of SHMOs by microbial fermentation, the microorganism is typically cultivated in the presence of exogenous sialic acid.

International Publications WO 2007/101862 A1 and WO 2014/153252 A1 disclose methods and compositions for engineering bacteria to produce sialylated oligosaccharides. However, fermentative production of 6'-sialyllactose is known to be associated by the biosynthesis of other carbohydrates such as disialyllactose (Drouillard, S. et al. (2010) Efficient synthesis of 6'-sialyllactose, 6,6'-disialyllactose, and 6'-KDO-lactose by metabolically engineered E. coli expressing a multifunctional sialyltransferase from the Photobacterium sp. JT-ISH-224. Carbohydrate Research 345: 1394-1399), which is generated from 6'-sialyllactose due to the activity of the sialyltransferase being expressed in the genetically engineered bacteria. In addition, autoclaving (heat treatment) carbohydrates (for example lactose) leads to the formation of undesired by-products such as aldol or maillard products, or rearrangement reactions (e.g. conversion of lactose to lactulose). Presence of such contaminants in the final product, in particular in larger amounts, is undesired or even inacceptable.

In addition, use of genetically engineered bacteria for producing oligosaccharides results in the contamination of the fermentation broth with recombinant material such as nucleic acid molecules or polypeptides resulting from the microorganism. However, contamination of a product for human consumption with recombinant DNA or proteins is neither acceptable by regulatory authorities nor by consumers today. Thus, any nucleic acids and proteins resulting from the recombinant microorganism need to be removed from the desired human milk oligosaccharide. Detection limits for recombinant DNA molecules are very low today which requires thorough purification schemes. In case of qPCR based detection of DNA, even as little as a single DNA molecule can be detected in a sample.

"State of the art" processes for purifying individual oligosaccharides from a fermentation broth are technically complex and often uneconomical, in particular when said oligosaccharide is intended for use in food applications. For the purification of the disaccharide lactose or sucrose from complex mixtures such as whey or molasses, industrial scale processes have been developed which involve multiple crystallisation steps. However, said methods are elaborate and only lead to low yields. Also, these processes are not applicable for rendering an oligosaccharide obtained by microbial fermentation suitable for use in the food industry, as such a fermentation broth - other than whey and molasses - is not already a food product.

However, filtration processes like ultrafiltration microfiltration and nanofiltration are technically easy to perform, if all process parameters are known and optimized. Diafiltration is another suitable process that involves the addition of fresh water to a solution in order to remove membrane permeable components. Ultrafiltration and microfiltration are usually used for separating much larger molecules like proteins or cells from a fermentation broth or a aqueous solution. Additionally, the removal of water, minerals and very small molecules by nanofiltration is well known and used in dairy industry for the concentration and demineralization of whey. In most cases membrane materials for nano- and ultrafiltration base on materials like piperazine, polyamide, polyethersulfone, polyethylene glycol and ceramic. Membranes can be assembled e.g. as hollow fibre modules, spiral wound modules and flat sheet membranes and the assembly can lead to different separation performances. In general, nanofiltration membranes possess a molecular weight cut-off in the range of 150-300 Daltons. Membranes with a molecular weight cut-off in the range of 400-600 Daltons are very rare, especially for the large-scale industrial production. This makes the separation of oligosaccharides still complex because other separation techniques like a chromatography, either batch-wise or continuously, are necessary.

United States Patent Application Publication No. US 2014/0170293 A1 concerns a concentrate derived from milk or a milk product comprising sialyllactose in amounts higher than the normal amounts found in milk or milk product, and a process for preparation of such a concentrate by ultrafiltration and diafiltration using a thin film polyamide based membrane.

Chinese laid open publication CN 103788144 A relates to a method of extracting ganglioside GM1 from fresh pig brain, wherein a primary extract is subjected to ultrafiltration and nanofiltration.

European Patent Application EP 2 484 686 A1 discloses a method for separating a sialyllactose from milk material, wherein the method comprises removing proteins from the milk material, adjusting the pH of the thus obtained protein-free liquid, and concentrating the pH-adjusted liquid by an ultrafiltration membrane.

International Publication No. WO 2017/220697 A1 pertains to a process for the production of a nutritional product containing milk protein and milk saccharide. The process comprises subjecting a milk feed to microfiltration or microfiltration/diafiltration to obtain a retentate enriched with respect to micellar casein and a permeate enriched with respect to milk serum protein, subjecting the permeate to nanofiltration or nanofiltration/ultrafiltration, and subjecting the retentate fo the nanofiltration to electrodialysis.

Whereas gel filtration chromatography is a convenient lab scale method only simulated moving bed chromatography represents a suitable method for purification of 6-SL at scales compatible to food production. However, all other published processes suffer from the problem that contaminating carbohydrates cannot be effectively removed from the desired product.

It was therefore an object of the present invention to provide a simple and costefficient method for the purification of a sialyllactose from microbial fermentations, wherein nucleic acids and polypeptides originating from the recombinant microorganism can be removed from the desired oligosaccharide as well as said other carbohydrates.

### Summary

Provided is a method for the purification of a sialyllactose which is simple, costefficient and scalable. The method for the purification of a sialyllactose can be performed without the use of an organic solvent for crystallization of said sialyllactose. In addition, the method for the purification of a sialyllactose can be performed without a discontinuous chromatographic step.

Sialyllactoses represent human milk oligosaccharides which inclusion into infant formula and medical food is highly desirable. The high cost of sialyllactoses prevents its wide spread use in particular in infant formula. In particular the purification of products from microbial fermentation are often elaborate and expensive. Also, the use of organic solvents and discontinuous chromatographic steps make 6'-sialyllactose and other neutral oligosaccharides prohibitively expensive. Also, the use of ethanol is not acceptable by certain religious food standards, for example Halal. Thus, the invention relates to a simple cost effective method to purify a sialyllactose from microbial fermentation using an recombinant processing aid, yielding a sialyllactose product suitable for human consumption, in particular suitable for infant food and medical nutrition products.

The purification process is based on the use of two different membranes for the purification/separation of a sialyllactose from contaminating carbohydrates by means of filtration and comprises two steps of membrane filtration, wherein one membrane has a molecular weight cut-off of between about 300 and about 500 Dalton, and wherein the other membrane has a molecular weight cut-off of between about 600 to about 800 Dalton.

### Detailed description

Provided is a method for the purification of a sialyllactose from other carbohydrates, wherein the method comprises the steps of subjecting an aqueous solution containing a sialyllactose and said other carbohydrates to two membrane filtration steps using different membranes, wherein one membrane has a molecular weight cut-off of between about 300 to about 500 Dalton, and wherein the other membrane has a molecular weight cut-off of between about 600 to about 800 Dalton.

The membrane having a molecular weight cut-off of between about 300 to about 500 Dalton allows removal of the bulk of carbohydrates having a molecular weight that is smaller than that of a sialyllactose. Upon filtration, SL and carbohydrates having a molecular weight larger than that of SL are retained in the retentate.

In an additional and/or alternative embodiment, the membrane having a molecular weight cut-off of between about 300 to about 500 Dalton has a pore size of 1 to 2 nm.

Suitable membranes having a molecular weight cut-off that is smaller than the molecular weight of SL are - for example - TriSep XN-45 (TriSep Corporation, USA), Dairy DK (Suez Water Technologies, formerly GE) and Filmtech NF270 (Dow).

The membrane having a molecular weight cut-off of between about 600 to about 800 Dalton possesses permeability for sialyllactose and carbohydrates having a molecular weight smaller than that of SL. Upon filtration, SL is present in the filtrate whereas carbohydrates having a molecular weight larger than that of SL remain in the retentate.

In an additional and/or alternative embodiment, the membrane having a molecular weight cut-off of between about 600 to about 800 Dalton has a pore size of 2.5 to 3 nm.

Suitable membranes for possessing permeability for 6-SL and retaining carbohydrates having a molecular weight larger than that of 6-SL in the retentate are - for example - TangenX SIUS TFF 0.65kDa membrane (Repligen Corporation), Zirkonia modules 3nm (Pervatech BV) and S-CUT YSNF-YS600 (CUT/Bürkert).

The method circumvents the use of expensive discontinuous chromatographic steps and also renders precipitation or crystallisation steps using organic solvents unnecessary. Thus, in an additional and/or alternative embodiment, the method does not comprise one or more discontinuous chromatography steps and/or one or more steps of precipitating and/or crystallizing 6-SL by using an organic solvent.

In an additional and/or alternative embodiment, the aqueous solution is obtained from a fermentation or enzymatic process for the production of a sialyllactose.

The method described herein is suitable for the purification of the human milk oligosaccharides 3'-sialyllactose or 6'-sialyllactose from a microbial fermentation or biocatalysis reaction in multi-ton amounts, because it is economically feasible and scalable.

In an additional and/or alternative embodiment, the aqueous solution is obtained from a fermentation, i.e. cultivating microbial cells that are able to produce a sialyllactose in a culture medium (fermentation broth) and under conditions that are permissive for the microbial cells to produce the sialyllactose, by separating the biomass from the fermentation broth. Preferably, separating the biomass from the fermentation broth comprises at least one step of ultrafiltration, preferably two steps of ultrafiltration, more preferably a first ultrafiltration using a membrane having a molecular weight cut-off of about 500 kDa and a second ultrafiltration using a membrane having a molecular weight cut-off of about 150 kDa.

In an additional and/or alternative embodiment, the aqueous solution is treated with a cation exchange resin, preferably in H⁺ form, and with an anion exchange resin, preferably in Cl⁻ form. Preferably, the aqueous solution containing the sialyllactose and other carbohydrates is treated with ion exchange resins before being subjected to the membrane filtration steps for removal of other carbohydrates.

In an additional and/or alternative embodiment, the method further comprises a step of dialysis, preferably a step of electrodialysis, for removal of ions. Preferably, the aqueous solution containing the sialyllactose is subjected to dialysis and/or electrodialysis after said other carbohydrates have been removed from the aqueous solution.

The product can be most conveniently supplied as a sterile concentrate or as a spray-dried product.

The method allows purifying 3'-sialyllactose or 6'-sialyllactose, i.e. separating 3-SL or 6-SL from other carbohydrates, wherein the purity of the sialyllactose in the aqueous solution is ≤ 70 %, ≤ 60 %, ≤ 50 %, ≤ 40 %, ≤ 30 %, ≤ 20 %; ≤ 10 % or ≤ 5 % prior to the purification and/or the aqueous solution contains the sialyllactose at a purity of ≥ 80 %, preferably of ≥ 85 % or more preferably ≥ 90 % after the purification.

In an additional and/or alternative embodiment, the purification comprises the following steps:
i.) separating the biomass from the fermentation broth;
ii.) subjecting the cell-free fermentation broth to at least one ion-exchange resin treatment for the removal of charged material, preferably to an anionexchanges resin treatment and a cation-exchange resin treatment;
iii.) subjecting the aqueous solution obtained in step ii. to a membrane filtration step using a membrane having a molecular weight cut-off of about 300 to about 500 Dalton to remove carbohydrates having a molecular weight smaller than that of a sialyllactose;
iv.) subjecting the retentate obtained in step iii. to a membrane filtration step using a membrane having a molecular weight cut-off of about 600 to about 800 Dalton to remove carbohydrates having a molecular weight larger than that of a sialyllactose; and - optionally -
v.) increasing the concentration of the sialyllactose present in the filtrate of step iv.

Whereas other procedures used for the purification of sialyllactose are rather complex and therefore expensive the method described herein before relies mainly on the use of two membrane filtration steps.

Certain embodiments comprise one or more further steps, such as dialysis steps (for the removal of salts), electrodialysis (for the removal of charged molecules), activated charcoal treatment (for the decolorization of the product solution) and/or other filtration processes (like endotoxin removal and/or sterile filtration). Although not necessary, the process may comprise treatment of the aqueous solution containing the sialyllactose with an organic solvent (such as short chain alcohols like methanol) for the precipitation of contaminating oligosaccharides or for elution after adsorption to activated charcoal with short chain alcohols and water mixtures, and/or for crystallisation of the sialyllactose.

The aqueous solution resulting from the method contains the sialyllactose, but no nucleic acids and polypeptides of the microbial cells. In addition, said aqueous solution hardly - if at all - contains monosaccharides and/or disialyllactose.

The present invention will be described with respect to particular embodiments, but the invention is not limited thereto but only by the claims. Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description and drawings provided herein, numerous specific details are set forth. However, it is understood that embodiments of the invention may be practiced without these specific details. In other instances, well-known methods, structures and techniques have not been shown in detail in order not to obscure an understanding of this description.

The invention will now be described by a detailed description of several embodiments of the invention. It is clear that other embodiments of the invention can be configured according to the knowledge of persons skilled in the art without departing from the true spirit or technical teaching of the invention, the invention being limited only by the terms of the appended claims.

### Examples

### Example 1. Fermentative production of 6'-sialyllactose

A 6'-sialyllactose feed-batch fermentation employing a recombinant 6'-sialyllactose synthesizing *E. coli* strain (*E. coli* BL21(DE3) *ΔlacZ*) expressing the α-2,6-sialyltransferase gene *plsT6* from *Photobacterium leiognathi* JT-SHIZ-119 was performed. To enhance the biosynthesis of CMP-sialic acid, genes encoding the glucosamine-6-phosphate synthase GlmS from *E. coli,* the N-acetylglucosamine 2-epimerase Slr1975 from *Synechocystis sp.,* the glucosamine 6-phosphat N-acetyltransferase Gna1 from *Saccharomyces cerevisiae,* the phosphoenolpyruvate synthase PpsA from *E. coli,* the N-acetylneuraminate synthase NeuB, and the CMP-sialic acid synthetase NeuA, the latter both from *Campylobacter jejuni,* were chromosomally integrated into the *E. coli* BL21(DE3) host. Furthermore, the gene encoding the lactose permease LacY from *E. coli,* and the genes *cscB* (sucrose permease), cscK (fructokinase), *cscA* (sucrose hydrolase), and *cscR* (transcriptional regulator) from *E. coli* W were integrated into the BL21 genome such that transcription of the integrated genes is initiated from constitutive promotors, either from the tetracycline promotor Pₜₑₜ or the PT5 promotor. A functional gal-operon consisting of and expression the genes *galE* (UDP-glucose-4-epimerase), *galT* (galactose-1-phosphate uridylyltransferase), *galK* (galactokinase), and *galM* (galactose-1-epimerase) was transferred from *E. coli* K12 to the genome of the BL21 strain. To prevent degradation of N-acetylglucosamine 6-phosphate, genes coding for N-acetylglucosamine-6-phosphate deacetylase (NagA), glucosamine-6-phosphate deaminase (NagB), and the N-acetylglucosamine specific PTS protein IIABC (NagE) were deleted from the bacterial chromosome. Additionally, the operon *manXYZ,* encoding a sugar transporter of the *E. coli* PTS system for mannose, glucose, glucosamine and N-acetylglucosamine, was deleted, as well as the genes *nanA, nanK, nanE,* and *nanT,* encoding the N-acetylneuraminate lyase, the N-acetylmannosamine kinase, the N-acetylmannosamine-6-phosphate epimerase, and the sialic acid transporter, respectively. The gene encoding the *N-*acetylgalactosamine-6-phosphate deacetylase (AgaA) was also deleted.

The 6'-sialyllactose producing *E. coli* strain was grown in a defined mineral salts medium, comprising 7 g·l⁻¹ NH₄H₂PO₄, 7 g·l⁻¹ K₂HPO₄, 2 g·l⁻¹ KOH, 0.3g·l⁻¹ citric acid, 5 g·l⁻¹ NH₄Cl, 1 ml·l⁻¹ antifoam (Struktol J673, Schill + Seilacher), 0.1 mM CaCI2, 8 mM MgSO₄, trace-elements and 2% sucrose as carbon source. Trace elements consisted of 0.101 g·l⁻¹ nitrilotriacetic acid, pH 6.5, 0.056 g·l⁻¹ ammonium ferric citrate, 0.01 g·l⁻¹ MnCl₂ × 4 H₂O, 0.002 g·l⁻¹ CoCl₂ × 6 H₂O, 0.001 g·l⁻¹ CuCl₂ × 2 H₂O, 0.002 g·l⁻¹ boric acid, 0.009 g·l⁻¹ ZnSO₄ × 7 H₂O, 0.001 g·l⁻¹ Na₂MoO₄ × 2 H₂O, 0.002 g·l⁻¹ Na₂SeO₃, 0.002 g·l⁻¹ NiSO₄ × 6 H₂O.

The sucrose feed (500 g·l⁻¹) was supplemented with 8 mM MgSO₄, 0.1 mM CaCl₂, trace elements, and 5 g·l⁻¹ NH₄Cl. For 6'-sialyllactose formation, a lactose feed of 216 g·l⁻¹ was employed. The pH of the culture medium was controlled by using ammonia solution (25% v/v). Fed batch fermentation was conducted at 30°C under constant aeration and agitation for 72 hours by applying a sucrose feeding rate of 5.5 - 7 ml·l⁻¹·h⁻¹, referring to the starting volume. At 72 hours after the start of the fermentation, most of the added lactose was converted into 6'-sialyllactose. In order to remove residual lactose in the fermentation supernatant, β-galactosidase was added to the fermentation vessel.

### Example 2: Preparation of a cell free fermentation broth

The cell-mass (about 10% of the fermentation broth) was separated from the medium by ultrafiltration (0.05 µm cut-off) (CUT membrane technology, Erkrath, Germany) followed by a cross-flow filtrations using a filter having a MWCO of 150 kDa (Microdyn-Nadir, Wiesbaden, Germany).

### Example 3: Ion exchange resin treatment of a 6'-sialyllactose-containing cell free fermentation broth

The cell-free fermentation broth as obtained from example 2 contained 6'-sialyllactose (at a purity of 9 % by dry weight) in a volume of 1000 liters and was passed over a strong cationic ion exchange resin (Lewatit^{®} S2568 obtained from the company Lanxess, Germany) in H⁺ form in order to remove positive charged contaminants (size of the ion exchanger bed volume was 200 L). The elution of 6-SL from the ion exchange resin was continued with deionized water. The obtained solution was then set to pH 7 by the addition of sodium hydroxide solution. The solution was then (without delay) passed over an anionic ion exchanger column (bed volume of the ion exchanger was 200 L). The used strong anionic ion exchanger Lewatit^{®} S6368 A (Lanxess, Germany) was in the chloride (Cl⁻) form. The elution of 6-SL was continued with deionized water. The obtained solution was again neutralized to pH 7.

### Example 4: First filtration for the removal of smaller molecules/carbohydrates

The solution obtained by ion exchange resin treatment set forth in example 3 was diafiltrated using a TriSep XN-45 (TriSep Corporation, USA) nanofiltration membrane and 500 L of deionized water. The resulting solution was further concentrated using the nanofiltration membrane (RE 8040-BE, CSM-Saehan) wherein a 6'-sialyllactose solution with a purity of 31 % (by dry weight) and a conductivity of 8 mS/cm was obtained.

### Example 5: Second filtration for the removal of larger molecules/carbohydrates

To separate the 6'-sialyllactose from fermentative produced by-product disialyllactose a second membrane treatment was employed. A TangenX SIUS TFF membrane with a nominal cut-off 0.65kDa (Repligen Corporation) was used for filtration. The solution containing 94.4% 6'-sialyllactose and 6.6% disialyllactose (ratio 15:1) was cross-flow filtered with the described system and the concentration of 6'-sialyllactose and disialyllactose was determinate inside permeate and retentate. The results show a significant reduction of disialyllactose amount inside the permeate from 6.6% to 0.2% (ratio 6'-sialyllactose to disialyllactose was 55:1) whereas the ratio of 6'-sialyllactose to disialyllactose inside the retentate was 14:1. This result shows that the 6'-sialyllactose could be separated from the disialyllactose by using an additional filtration step. This observation could be also shown by using a second kind of membrane with a similar cut-off from a different supplier. By using a S-CUT YSNF-YS600-2540-M46D-ATD membrane (CUT Membrane Technology GmbH) with a nominal cut-off of 600-800 Da the 6'-sialyllactose was also separated from disialyllactose by cross-flow filtration.

### Example 6: Electrodialysis treatment

The permeate obtained in example 5 was electrodialysed to 2 mS/cm using a PC-Cell 15 electrodialysis apparatus (PC-Cell, Heusweiler, Germany) equipped with an PC-Cell ED 1000H membrane stack. This membrane stack was equipped with the following membranes: cation exchange membrane CEM: PK SK and the anion exchange membrane AEM:PcAcid60 with an size exclusion limit of 60 Da. A 0.025 M sulfamic acid (amidosulfonic acid) solution was used as an electrolyte in the electrodialysis process. After electrodialysis a solution containing 6'-sialyllactose at a purity of 83 % (by dry weight) was obtained. Alternatively to the electrodialysis treatment, a diafiltration process could be employed.

### Example 7: Concentration and activated carbon treatment.

Some water was removed from the aqueous solution using a reversed osmosis filter and rotary evaporation at 45 °C to obtain a 25 % (m/v) 6'-sialyllactose solution. The resulting aqueous solution was then treated with activated carbon (Norit SA2). 125 g activated carbon was employed for 1 L 25 % (m/v) 6'-sialyllactose.

### Example 8: Sterile filtration and endotoxin removal

Subsequently, the solution obtained in example 7 was subjected to sterile filtration by passing the solution through a 6 kDa filter (Pall Microza ultrafiltration hollow fiber module SEP-2013, Pall Corporation, Dreieich, Germany). The sterile solution was then stored at RT until spray-drying.

### Example 9: Spray-drying of 6'-sialyllactose

The so obtained (example 8) sterile 6'-sialyllactose were then spray-dried using a NUBILOSA LTC-GMP spray dryer (NUBILOSA, Konstanz, Germany). For the spray-drying of the 6'-sialyllactose the solution was passed under pressure with 3.5 bar through the spray dryer nozzles set to 130°C and flow was adjusted to maintaining an exhaust temperature between 67°C to 69°C. Using these settings a spray-dried powder with less than 9 % moisture could be obtained. The moisture contents were determined by Karl-Fischer titration.

### Example 10: LC-MS/MS analysis of 6'-sialyllactose

For quantification mass spectrometry analysis was performed by MRM (multiple reaction monitoring) using a LC Triple-Quadrupole MS detection system. Precursor ions are selected and analyzed in quadrupole 1, fragmentation takes place in the collision cell using argon as CID gas, selection of fragment ions is performed in quadrupole 3. Chromatographic separation of saccharides was performed on a XBridge Amide HPLC column (3.5 µm, 2.1 × 50 mm (Waters, USA) with a XBridge Amide guard cartridge (3.5 µm, 2.1 × 10 mm) (Waters, USA). Column oven temperature of the HPLC system was 50°C. The mobile phase was composed of acetonitrile/H₂O with 10 mM ammonium acetate. A 1 µl sample was injected into the instrument; the run was performed for 3.60 min with a flow rate of 400 µl/min. 6'sialyllactose was analyzed by MRM in ESI positive ionization mode. The mass spectrometer was operated at unit resolution. Sialyllactose forms an ion of m/z 656.2 [M+Na]. The precursor ion of sialyllactose was further fragmented in the collision cell into the fragment ions m/z 612.15, m/z 365.15 and m/z 314.15. Collision energy, Q1 and Q3 Pre Bias were optimized for each analyte individually. Quantification methods were established using commercially available standards (Carbosynth, Compton, UK).

## Claims

1. A method for the purification of a sialyllactose from other carbohydrates, **characterized in that** the method comprises the steps of subjecting an aqueous solution containing the sialyllactose and other carbohydrates to two membrane filtration steps using different membranes, wherein
- one membrane has a molecular weight cut-off of between about 300 to about 500 Dalton, and
- the other membrane has a molecular weight cut-off of between about 600 to about 800 Dalton.

2. The method according to claim 1, wherein the aqueous solution is obtained from a fermentation or enzymatic process for the production of the sialyllactose.

3. The method according to claim 1 or 2, wherein the aqueous solution is obtained from a fermentation by separating the biomass from the fermentation broth.

4. The method according to claim 3, wherein separating the biomass from the fermentation broth comprises at least one step of ultrafiltration, preferably two steps of ultrafiltration, more preferably a first ultrafiltration using a membrane having a molecular weight cut-off of about 500 kDa and a second ultrafiltration using a membrane having a molecular weight cut-off of about 150 kDa.

5. The method according to any one of claims 1 to 4, wherein the aqueous solution containing the sialyllactose is treated with a cation exchange resin, preferably in H⁺ form, and an anion exchange resin, preferably in Cl⁻ form, more preferably before subjecting the aqueous solution to the membrane filtration steps.

6. The method according to any one of claims 1 to 5, wherein the method further comprises a step of dialysis, preferably of electrodialysis.

7. The method according to any one of claims 1 to 6, wherein the purity of sialyllactose in the aqueous solution is ≤ 70 %, ≤ 60 %, ≤ 50 %, ≤ 40 %, ≤ 30 %, ≤ 20 %; ≤ 10 % or ≤ 5 % prior to the purification and /or the aqueous solution contains the sialyllactose at a purity of ≥ 80 %, preferably of ≥ 85 % or more preferably ≥ 90 % after the purification.

8. The method according to any one of claims 1 to 7, wherein the sialyllactose is 3'-sialyllactose or 6'-sialyllactose.

## Patentansprüche

1. Ein Verfahren zur Reinigung einer Siallyllactose von anderen Kohlenhydraten, **dadurch gekennzeichnet, dass** das Verfahren die Schritte des Zuführens einer wässrigen Lösung, die die Sialyllactose und andere Kohlenhydrate enthält, zu zwei Membranfiltrationsschritten mit unterschiedlichen Membranen umfasst, wobei
- eine Membran einen Molekulargewichtsausschluss von zwischen etwa 300 bis etwa 500 Dalton aufweist, und
- die andere Membran einen Molekulargewichtsausschluss von zwischen etwa 600 bis etwa 800 dalton aufweist.

2. Das Verfahren gemäß Anspruch 1, wobei die wässrige Lösung aus einer Fermentation oder einem enzymatischen Verfahren zur Herstellung der Sialyllactose erhalten wird.

3. Das Verfahren gemäß Anspruch 1 oder 2, wobei die wässrige Lösung aus seiner Fermentation durch Abtrennen der Biomasse von dem Fermentationsmedium erhalten wird.

4. Das Verfahren gemäß Anspruch 3, wobei das Abtrennen der Biomasse vom Fermentationsmedium mindestens einen Schritt der Ultrafiltration aufweist, vorzugsweise zwei Ultrafiltrationsschritte, besonders bevorzugt einer ersten Ultrafiltration mit einer Membran, die einen Molekulargewichtsausschluss von etwa 500 kDa hat, und einer zweiten Ultrafiltration mit einer Membran, die einen Molekulargewichtsausschluss von etwa 150 kDa hat.

5. Das Verfahren gemäß einem der Ansprüche 1 bis 4, wobei die wässrige Lösung, die die Sialyllactose enthält, mit einem Kationenaustauscherharz, vorzugsweise in H⁺-Form, behandelt wird, und einem Anionenaustauscherharz, vorzugsweise in Cl⁻-Form, besonders bevorzugt bevor die wässrige Lösung den Membranfiltrationsschrtten zugeführt wird.

6. Das Verfahren gemäß einem der Ansprüche 1 bis 5, wobei das Verfahren zusätzlich einen Schritt der Dialyse umfasst, vorzugsweise der Elektrodialyse.

7. Das Verfahren gemäß einem der Ansprüche 1 bis 6, wobei die Reinheit der wässrigen Lösung ≤ 70 %, ≤ 60 %, ≤ 50 %, ≤ 40 %, ≤ 30 %, ≤ 20 % ; ≤ 10 % oder ≤ 5 % vor der Reinigung beträgt und/oder die wässrige Lösung die Sialyllactose in einer Reinheit von ≥ 80 %, vorzugsweise ≥ 85 % oder besonders bevorzugt ≥ 90 % nach der Reinigung enthält.

8. Das Verfahren gemäß einem der Ansprüche 1 bis 7, wobei die Sialyllactose 3'-Sialyllactose oder 6'-Sialyllactose ist.

## Revendications

1. Un procédé de purification d'un sialyllactose à partir d'autres hydrates de carbone, **se caractérisant par** une méthode comprenant des étapes consistant à soumettre une solution aqueuse contenant le sialyllactose et d'autres hydrates de carbone à deux étapes de filtration par membrane, en utilisant des membranes différentes, où
- l'une des membranes a un seuil de coupure de poids moléculaire compris entre 300 et env. 500 daltons, et
- l'autre membrane a un seuil de coupure de poids moléculaire compris entre 600 et env. 800 daltons.

2. Le procédé selon la revendication 1, où la solution aqueuse est obtenue par une méthode de fermentation ou enzymatique pour la production du sialyllactose.

3. Le procédé selon la revendication 1 ou 2, où la solution aqueuse est obtenue à partir d'une fermentation en séparant la biomasse du milieu de fermentation.

4. Le procédé selon la revendication 3, où la séparation de la biomasse du milieu de fermentation comprend au moins une étape d'ultrafiltration, plutôt deux étapes d'ultrafiltration, de préférence une première ultrafiltration utilisant une membrane ayant un seuil de coupure de poids moléculaire d'environ 500 kDa et une seconde ultrafiltration utilisant une membrane ayant un seuil de coupure de poids moléculaire d'environ 150 kDa.

5. Le procédé selon l'une des revendications 1 à 4, où la solution aqueuse contenant le sialyllactose est traitée avec une résine échangeuse de cations, plutôt sous forme H⁺, et une résine échangeuse d'anions, plutôt sous forme Cl⁻, de préférence avant de soumettre la solution aqueuse aux étapes de filtration avec membrane.

6. Le procédé selon l'une des revendications 1 à 5, où le procédé comprend en outre une étape de dialyse, de préférence d'électrodialyse.

7. Le procédé selon l'une des revendications 1 à 6, où la pureté du sialyllactose dans la solution aqueuse est ≤ 70 %, ≤ 60 %, ≤ 50 %, ≤ 40 %, ≤ 30 %, ≤ 20 % ; ≤ 10 % ou ≤ 5 % avant la purification et /ou la solution aqueuse contient le sialyllactose à une pureté ≥ 80 %, plutôt ≥ 85 % ou de préférence ≥ 90 % après la purification.

8. Le procédé selon l'une des revendications 1 à 7, où le sialyllactose est le 3'sialyllactose ou le 6'-sialyllactose.
